## Europäisches Patentamt

## European Patent Office

## Office européen des brevets

(19)

(11) Publication number: **0 046 178**
A2

(12) # EUROPEAN PATENT APPLICATION

(21) Application number: **81105112.7**

(51) Int. Cl.³: **G 01 N 31/00**

(22) Date of filing: **01.07.81**

(30) Priority: **20.08.80 US 179855**

(71) Applicant: **THE PERKIN-ELMER CORPORATION, Main Avenue, Norwalk Connecticut 06432 (US)**

(43) Date of publication of application: **24.02.82 Bulletin 82/8**

(72) Inventor: **Culmo, Robert F., 62 Hallsey Lane, Woodbridge Connecticut 06525 (US)**

(74) Representative: **Patentanwälte Grünecker, Dr.Kinkeldey Dr.Stockmair, Dr.Schumann,Jakob, Dr.Bezold Meister, Hilgers, Dr.Meyer-Plath, Maximilianstrasse 43, D-8000 München 22 (DE)**

(84) Designated Contracting States: **DE GB IT**

(54) **Apparatus useful in determining organic carbon content of a sample.**

(57) An apparatus used in determining the organic carbon content of a sample material includes a gas passage having a reaction vessel associated therewith. The passage is in gaseous serial communication with an inlet passage of a single channel elemental analyzer.

EP 0 046 178 A2

ACTORUM AG

APPARATUS USEFUL IN DETERMINING ORGANIC CARBON CONTENT OF A SAMPLE

BACKGROUND OF THE INVENTION

The present invention generally relates to the determination of the organic carbon content of a sample material and, in particular, relates to an apparatus useful in making such a determination in cooperation with a single channel elemental analyzer.

The presence of organic carbon in various materials and the relative quantity thereof is of great interest in many fields. For example, in the energy field the quantity of organic carbon in a given material is indicative of the usefulness of that material as a source of fuel. In this field the determination of the organic carbon content of a sample of oil is a prime example. Further, in the field of environmental science the presence and quantity of organic carbon is an indicator of potential sources of food as well as an indication of pollution levels.

Unfortunately, in materials of interest, particularly in the energy and environmental fields, carbon is almost always present in both organic and inorganic form. Thus, in order to determine the presence and quantity of only the organic carbon, the presence of the inorganic carbon must be thoroughly considered.

Presently there exists a method whereby a sample is pretreated with an acid to drive off the inorganic carbon. The sample is then dried and the organic carbon content is determined from the residue. However, this procedure carries with it the inherent danger of either evolving some of the organic carbon with the inorganic carbon or not completely evolving the inorganic carbon. In either event the results can be inaccurate.

Another conventional method of determining the organic carbon content of a sample is to first measure the total carbon content of a portion of the sample and then measure the inorganic carbon content using a second portion of the sample. The difference between the two measurements then yields the organic carbon content of the sample material. Presently, however, this method and the apparatus employed therefor are designed for analyzing only liquid samples. In addition, these measurements are rather slow and also require an elaborate apparatus as well as a skilled operator.

Hence, at present there is a need for an apparatus useful for the determination of the organic carbon content of a solid sample which is not only accurate but also relatively straightforward to implement.

## SUMMARY OF THE INVENTION

In view of the foregoing it is a primary object of the present invention to provide an apparatus for use in determining the organic carbon content of a sample. This is accomplished generally by placing a gas passage means, having a reagent vessel associated therewith, in series with the channel of a single channel elemental analyzer.

This and other objects will become apparent from the following drawing and detailed description of an apparatus useful for determining the organic carbon content of a sample.

## - 3 -

BRIEF DESCRIPTION OF THE DRAWING

Figure 1 is a pictorial representation, not drawn to scale, of an apparatus embodying the principles of the present invention.

Figure 2 is a pictorial representation, not drawn to scale, of a gas passage means of an apparatus embodying the principals of the present invention.

DETAILED DESCRIPTION OF THE INVENTION

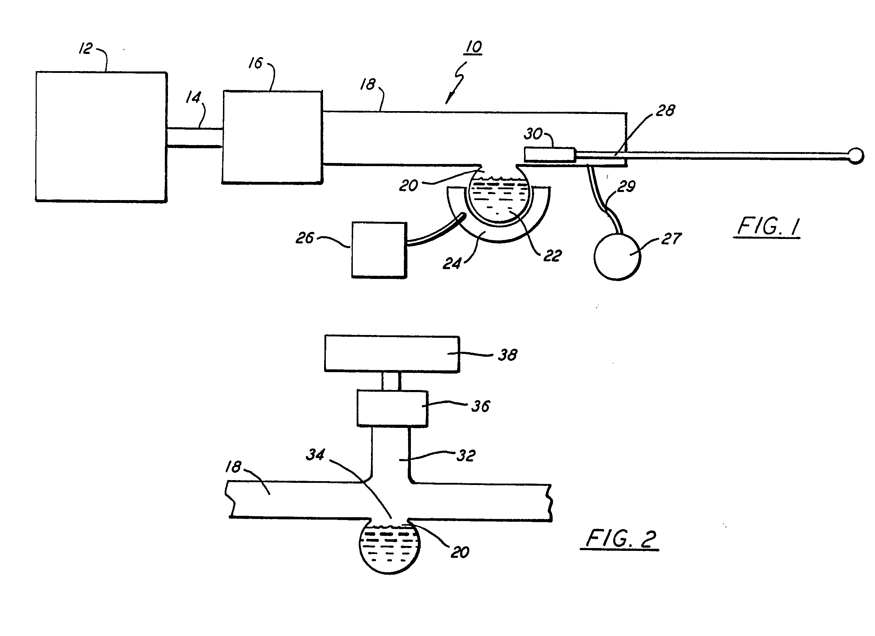

A system useful for determining the organic carbon content of a sample material is generally shown in Figure 1. As shown, the system, indicated generally in Figure 1 by the numeral 10, includes a single channel elemental analyzer 12 having a gas inlet passage 14 and a combustion tube 16 in serial gaseous communication with the inlet passage 14. The system 10 further includes an inorganic carbon gas passage means 18 in serial gaseous communication with the inlet passage 14 via the combustion tube 16. The means 18 has a reaction vessel 20 in gaseous communication therewith which vessel 20 is adapted to contain a non-oxidizing acidic fluid 22. The reaction vessel 20 preferably has a means 24 for heating the fluid 22 which can be placed therein, which means 24 is controlled by a heater control means 26. In addition the system includes a source 27 of pressurized inert gas and means 29 for delivering that gas to the passage 18.

The elemental analyzer 12 is characterized in that any gas which is to be analyzed thereby passes through the single inlet passage 14, i.e. it is a single channel analyzer. As well known in the instrument field, all gasses passing through the inlet passage 14 follow the same path without the analyzer 12. One such conventional elemental analyzer is the Model 240B Elemental Analyzer manufactured and sold by The Perkin-Elmer Corporation, Norwalk, Connecticut.

The combustion tube 16 is connected to the inlet passage 14 such that when a sample is oxidized therein the gas released thereby passes, i.e. it is carried by the inert gas flowing from the source 27, into the analyzer 12 via the inlet passage 14. Combustion tubes, such as that indicated at numeral 16, are well known in the elemental analysis art and for that reason further details of the structure thereof are not deemed necessary.

The inorganic carbon gas passage 18 is preferably a hollow quartz tube and has a reaction vessel 20 in gaseous communication therewith. Preferably the passage 18 is adapted with a means 28 for introducing a sample vial 30 into the reaction vessel 20. In the preferred embodiment the same means 28, which can be a slidably moveable rod, or ladle, can be used to introduce a boat, not shown in the drawing, containing a sample into the combustion tube 16. Such a ladle has a length which is designed to ensure its sliding movement over the opening 34 between the passage 18 and the reaction vessel 20 without the boat tipping or otherwise losing sample material.

To enhance the reaction between the sample in the vial 30 and the non-oxidizing acidic fluid 22, more fully discussed below, the reaction vessel 20 is provided with a means 24 for heating the fluid 22. The means 24 can be any form of heater known in the art and is controlled by a cooperative heater control means 26, also known in the art.

In operation the organic carbon content of a sample is determined as follows. The sample material is divided into at least two portions. Preferably the portions of the sample material are equal in weight.

As a first step, although the sequence is not restrictive, a

## - 5 -

first portion of the sample material is placed in the aforementioned boat and introduced into the combustion tube 16 wherein it is completely oxidized via known combustion methods. The gas therefrom is then carried by inert gas from the source 27 and enters the elemental analyzer 12 via inlet passage 14 and the total, i.e. both inorganic and organic carbon content, is quantified.

A second portion of the sample is then prepared for testing. The preferred method of preparation is to insert the second portion of the sample into a platinum vial 30 having one end open and the other end screened so as to prevent the rapid egress of the second portion therefrom but nevertheless allows the non-oxidizing acidic fluid 22 to easily penetrate the vial 30. In one embodiment the second portion of the sample is cushioned within the vial 30 by alumina ($Al_2O_3$) wool. In order to prevent the loss of available acid and/or the contamination of the evolved gas from the reaction, the vial 30 is preferably made of a material which is non-corrosive with respect to the non-oxidizing acidic fluid 22. Of course, the alumina wool is a ceramic and, as well known, is highly non-corrosive to most acids. In the preferred embodiment, the vial 30 is fabricated from platinum and the non-oxidizing acidic fluid 22 is phosphoric acid. Although other acids having non-oxidizing properties toward organic materials can be used, i.e. hydrochloric acid, phosphoric acid is preferred for a number of reasons. One reason is because it has a relatively high boiling point which permits its use at temperatures on the order of about $150^{\circ}$ C. By heating the acid 22 to such a temperature, complete decomposition of carbonates within the second portion of the sample material is achieved in a relatively short period of time, i.e. on the order of about one or two minutes. In addition, phosphoric acid is, as well known, a tri-basic acid, that is it has three replaceable hydrogen atoms, which is indicative of its comparative effectiveness to

- 6 -

other non-oxidizing acids on a per unit volume basis.

The vial 30 containing the second portion of the sample material is introduced to the reaction vessel 20 via the rod 28. The ensuing reaction between the second portion of the sample material and the non-oxidizing phosphoric acid 22 generates a gas, for example carbon dioxide, having as a constituent thereof the entire quantity of inorganic carbon contained in the second portion of the sample material.

The inorganic carbon containing gas is then carried by the inert gas from the source 27, via the combustion tube 16, which in this instance acts as a channel or a guide, through the inlet passage 14 and into the elemental analyzer 12. Another advantage of using a platinum vial 30 to introduce the second portion of the sample material into the acidic fluid 22 is that the vial 30 itself ensures that the sample material contained therein is completely submerged in the fluid 22. Hence the acidic fluid 22 attacks the entire surface of the second portion substantially uniformly. If the sample were less dense than the fluid and were introduced in a manner where the sample was not contained, then the sample would float on the surface and would take an indefinite time to react. Thus a more time uniform evolution of gas is produced.

The elemental analyzer 12, upon the introduction of the inorganic carbon containing gas, quantifies the inorganic carbon therein. Thus by subtracting the quantity of inorganic carbon from the previously determined quantity of the total carbon content of the sample the organic carbon content of the sample material can be determined.

It has been determined that during each reaction a small amount of water is driven off from the phosphoric acid 22. This water reduces the total effectiveness of the acid itself. In order to compensate for this water loss and to insure the total effectiveness of the acid a small drop, on the order of about 10 milligrams by weight, is preferably introduced into the sample vial 30 just prior to the vial 30 being introduced into the reaction vessel 20. This introduction of water, which substantially completely replaces that lost via the previous reaction, ensures that the phosphoric acid 22 maintains an effective uniform acidic strenth throughout a prolonged series of sample tests.

As in most analytical instrument operations, it would be extremely advantageous for the second portion of the sample to be introduced into the reaction vessel 20 by a remote mechanism or by an automated mechanism. Such an arrangement is depicted in Figure 2 wherein the inorganic carbon passage 18 is provided with a via inlet 32 extending from the passageway. For easy reference like numbers are used in the Figures to designate like elements of the apparatus shown. Preferably the vial inlet tube 32 is diametrically opposed to the opening 34 between the passageway 18 and the reaction vessel 20 and is adapted with a means 36 for gaseously sealing that tube during the actual reaction between the second portion of the sample and the acidic fluid in the reaction vessel 20. The act of sequentially releasing a series of vials into such a tube 32 and the mechanism associated therewith are extremely well known in the art or would be particularly straightforward to design. Thus, a particular mechanism is not shown in detail. Operationally, the vial delivery means 38 would, for example, on a preselected time sequence, first open the gaseous seal means 36 and introduce a vial 30 into the inlet tube 32 whereupon the means 38 would again seal the gaseous seal means 36.

The primary advantage of utilizing a system such as described herein is that both the total carbon content combustion procedure and the wet chemical inorganic gas carbon determining procedure are carried out by the same single channel elemental analyzer. The fact that all gasses are processed via the same channel increases the accuracy of the

## - 8 -

determination. This system and the apparatus associated therewith avoids many of the pitfalls presently existing in conventional technology such as utilization of two measurement means, each of which has its own errors, or such as the elimination of the need to rely on drying a material prior to determining the organic carbon content thereof. In addition, the present apparatus allows the analysis of solid samples whereas conventional techniques require the sample to be in a liquid state.

While the present invention has been described in relation to a particular embodiment of an apparatus for determining the organic carbon content of a sample, the above description is exemplary only and is not to be considered limiting to the spirit and scope of the present invention. The scope of the invention is to be determined from the appended claims and a reasonable interpretation thereof.

WHAT IS CLAIMED IS:

1. An apparatus for use in determining the organic carbon content in a solid sample; said apparatus comprising:

means for determining the quantity of total carbon content in a first portion of said sample; said determining means including a single channel elemental analyzer;

a passage means connected to the inlet of said analyzer;

a reaction vessel in gaseous communication with said passage means for containing a non-oxidizing acidic fluid;

means for introducing a second portion of said solid sample into said reaction vessel for reacting with said acidic fluid so that the inorganic carbon content therein evolves as a gas; and

means for passing said gas through said passage means to said inlet of said single channel elemental analyzer wherein said inorganic carbon content is quantified, whereby the organic carbon content of said sample is determinable by comparing the quantity of inorganic carbon with said total carbon quantity.

2. Apparatus as claimed in Claim 1 wherein said passage means is a hollow tube.

3. Apparatus as claimed in Claim 2 wherein said hollow tube is horizontal.

4. Apparatus as claimed in Claim 2 wherein said introducing means includes a vial, said vial being adapted so as to ensure submersion thereof in said acidic fluid, said vial being corrosive resistant to said acidic fluid; and

means for transporting said vial into said acidic fluid.

5. Apparatus as claimed in Claim 4 wherein said vial transporting means includes a vial pushing means cooperatively attached to a rod, said rod being slideably movable along said tube.

6. Apparatus as claimed in Claim 5 wherein said acidic fluid is phosphoric acid; and

said vial is platinum.

7. Apparatus as claimed in Claim 5 or 6 wherein said vial includes about 10 mg of water.

8. Apparatus as claimed in Claim 1 wherein said passage means and said reaction vessel are a unitary body.

9. Apparatus as claimed in Claim 1 further comprising:

means for heating said reaction vessel.

10. Apparatus as claimed in Claim 1 wherein said first portion of said sample and said second portion of said sample are equal in weight.

11. Apparatus as claimed in Claim 1 wherein said second sample is automatically provided to said reaction vessel.

FIG. 1

FIG. 2

1/1

CO46178